# EUROPEAN PATENT APPLICATION

(11) **EP 4 620 453 A1**
(43) Date of publication of application: **24.09.2025**
(21) Application number: 23891360.2
(22) Date of filing: 01.11.2023
(51) Int. Cl.: A61K 8/29, A61K 8/46, A61Q 1/00, A61Q 1/12, A61Q 17/04

(54) **AQUEOUS DISPERSION OF SURFACE-HYDROPHOBIZED INORGANIC POWDER AND COSMETIC PREPARATION CONTAINING SAME**

(30) Priority: 15.11.2022 JP 2022182769; 15.09.2023 JP 2023149732
(71) Applicant: Nikko Chemicals Co., Ltd., Tokyo 103-0002 (JP)
(72) Inventor: NAGATANI, Noboru, Tokyo 103-0002 (JP); NAKAMURA, Shohei, Tokyo 103-0002 (JP); NAKANO, Junya, Tokyo 103-0002 (JP)
(74) Representative: Hasegawa, Kan
(86) International application number: PCT/JP2023/039450
(87) International publication number: WO 2024/106207

(57) **Abstract**

To obtain a dispersion in which an inorganic powder with a hydrophobized surface is dispersed in water. The dispersion is easy to handle because the dispersion medium is water. The invention can be suitably used in a cosmetic where an inorganic powder with a hydrophobized surface is blended in an aqueous cosmetic. Provided are: a dispersion obtained by homogenously dispersing an inorganic powder with a hydrophobized surface in water, in which the inorganic powder with a hydrophobized surface can be stably dispersed in water by using a dialkyl sulfosuccinate or an alkyl sulfoacetate as a dispersant; and a cosmetic having high water resistance, providing a fresh feeling in use and obtained by using the aqueous dispersion of the inorganic-powder.

## Description

### TECHNICAL FIELD

The present invention relates to an aqueous dispersion of an inorganic powder with a hydrophobized surface and a cosmetic containing the same.

### BACKGROUND ART

Conventionally, when an inorganic powder is stably dispersed in water or an aqueous medium, a hydrophilic powder or a hydrophilically treated powder has been used. In contrast, when the hydrophobically treated powder is blended in water or an aqueous medium, a large amount of an ionic surfactant such as an alkyl phosphate, a polyoxyethylene-added alkyl ether phosphate, or lecithin was used as a dispersant, or a surfactant derived from a natural product (see Patent Literature 1) was used to disperse the powder in water. Furthermore, for improving dispersibility of a powder in water, a method of hydrophilically treating a powder, for example, the techniques disclosed in Patent Literatures 2 to 4 are known. Patent Literatures 2 and 3 disclose a technique related to coating treatment of a water-soluble cationic polymer and a low molecular-weight organosilicon derivative, whereas Patent Literature 4 discloses a technique of treating a surface with a polyether-modified silane. When such a hydrophilic powder is used, the hydrophilic powder can be easily added to a aqueous composition, and the amount thereof to be blended can be increased. The hydrophilic powder is advantageous since the characteristics of the powder are easily exhibited.

### Citation List

### Patent Literatures

Patent Literature 1: JP 2013-116864 A
Patent Literature 2: JP 4250551 B2
Patent Literature 3: JP 4475970 B2
Patent Literature 4: JP 2003-26958 A

### SUMMARY OF INVENTION

However, since the powder itself is hydrophilic, there is a problem in that it is difficult to obtain a coating film having sufficient water resistance as an inorganic-powder aqueous dispersion. When a large amount of a dispersant as mentioned above is blended as a means for blending the hydrophobically treated powder in water or an aqueous medium, it is also difficult to obtain a coating film having sufficient water resistance.

The invention has been made in view of these circumstances. An object of the invention is to provide an inorganic-powder aqueous dispersion having high dispersibility of an inorganic powder with a hydrophobizedsurface and high water resistance during use, and provide a cosmetic using the aqueous dispersion and having excellent feeling in use and water resistance.

The present inventors conducted intensive studies with a view to achieving the above object. As a result, they found that when a dialkyl sulfosuccinate and/or an alkyl sulfoacetate is used, an inorganic powder with a hydrophobizedsurface is stably dispersed in water, and that when the inorganic-powder aqueous dispersion is used, a cosmetic having high water resistance and satisfactory feeling(for example, freshness) in use can be obtained. Based on the finding, the invention was achieved.

To described more specific, the aqueous dispersion of the inorganic powder with a hydrophobized surface according to the invention contains the following components (A), (B), and (C).
(A) an inorganic powder with a hydrophobized surface
(B) a dialkylsulfosuccinate or an alkylsulfoacetate
(C) water.

The cosmetic of the invention is a cosmetic containing an aqueous dispersion of the inorganic powder with a hydrophobized surface according to the invention.

### DESCRIPTION OF EMBODIMENTS

Next, embodiments of an aqueous dispersion of an inorganic powder with a hydrophobized surface according to the invention and a cosmetic containing the dispersion will be described. Note that, in the specification, a range of "X to Y" means a range of "X or more and Y or less". Unless otherwise specified, operations are performed at room temperature (20°C to 30°C)/relative humidity of 45 to 55%RH. Further, it should be understood that all combinations of embodiments are disclosed in this application.

The aqueous dispersion of the inorganic powder with a hydrophobized surface according to the invention has high dispersibility of the inorganic powder with a hydrophobized surface and maintains the dispersibility even over time, and further has high water resistance. A cosmetic containing the aqueous dispersion has high water resistance and excellent fresh feeling in use. In addition, the cosmetic containing the aqueous dispersion of the inorganic powder with a hydrophobized surface according to the invention may be a cosmetic providing refreshing feeling without or less dry feeling or without or less powdery feeling.

### <Aqueous Dispersion>

### (Component (A): Inorganic powder with a hydrophobized surface)

Examples of the inorganic powder (base powder to be hydrophobically treated) to be used as component (A) include, but are not particularly limited to, commonly used powders such as zinc oxide, titanium oxide, cerium oxide, iron oxide, barium sulfate, calcium carbonate, silica, aluminum hydroxide, alumina, boron nitride, talc, mica, and kaolin. The inorganic powder is preferably at least one selected from the group consisting of titanium oxide, zinc oxide, iron oxide, cerium oxide, and composites of these, and particularly preferably an inorganic powder (hereinafter, also referred to as a UV-ray shielding particle) having ultraviolet ray absorbing/scattering ability (UV-ray shielding effect). The inorganic powder having an UV-ray shielding effect is particularly preferably at least one selected from the group consisting of zinc oxide, titanium oxide, and cerium oxide. In consideration that the inorganic powder having an UV-ray shielding effect is commonly used in cosmetic applications, the inorganic powder is also preferable since it is easily blended in an aqueous cosmetic. Furthermore, use of these inorganic powders is preferable since an inorganic thin film having an UV-ray shielding effect can be easily formed. Of them, zinc oxide and/or titanium oxide is particularly preferable as the inorganic powder. These inorganic powders may be used in combination of two or more types.

The inorganic powder to be used in the aqueous dispersion of the invention may be a composite powder prepared by coating the surface of the inorganic powder with another inorganic material. The inorganic material herein may be a commonly known inorganic surface treatment material. Examples thereof include zinc oxide, titanium oxide, cerium oxide, iron oxide, barium sulfate, hydrous silicic acid, silica, aluminum hydroxide and alumina. The amount of the inorganic coating material is preferably 1 to 25 mass% relative to the entire inorganic powder.

The average particle diameter (hereinafter, a simple term of particle diameter in the specification refers to an average particle diameter) of the inorganic powder is preferably 1 to 200 nm, more preferably 10 to 200 nm, and may be 1 to 100 nm. In particular, in the case of an UV-ray shielding particle, it is preferable to have the average particle diameter as mentioned above. Such a UV-ray shielding particle having such a particle diameter is particularly preferable because it has high visible light transparency and suitable ultraviolet ray shielding ability. When the inorganic powder is not a UV-ray shielding particle or requires no ultraviolet ray shielding ability, the particle diameter may have an optimum size depending on the use. Note that the particle diameter of the inorganic powder is determined by measuring the particle diameters of 200 particles randomly selected under an electron microscope and calculating an average of the primary particle diameters.

The shape of the inorganic powder is not particularly limited, and any shape such as a spherical shape, a rod shape, a needle shape, a spindle shape, or a plate shape can be used. In the case of rod-shaped, needle-shaped, or spindle-shaped particles, the average particle diameter thereof is defined as an average of the lengths of the minor axis, whereas in the case of plate-shaped particles, the average particle diameter thereof is defined as an average of the diagonal lengths of the surfaces.

The method for hydrophobically treating the surface of the inorganic powder is not particularly limited, and a commonly known method can be used. The hydrophobic treatment of a surface is a treatment for reducing the affinity of an inorganic powder surface to water. A surface treatment to be applied, after the hydrophobic treatment, with a material easily dissolved in water or a material dispersed in water does not correspond to the "hydrophobic treatment of a surface" according to the invention.

Examples of the hydrophobic treatment of a surface include a treatment with a surface treatment agent such as silicone oil, a fatty acid, or an alkoxysilane (for example, an alkylalkoxysilane). These surface treatments may be performed alone or in combination. According to a preferred embodiment of the invention, the inorganic powder with a hydrophobized surface is an inorganic powder treated with at least one selected from the group consisting of silicone oil, a fatty acid, and an alkoxysilane.

Examples of the silicone oil include so-called straight silicone oils such as dimethylpolysiloxane, methylphenylpolysiloxane, and methylhydrogenpolysiloxane (hydrogen dimethicone); and so-called branched silicone oils such as triethoxysilylethyl polydimethylsiloxyethyl dimethicone. Examples of the fatty acid include stearic acid, isostearic acid, lauric acid, myristic acid, and palmitic acid. As the alkoxysilane, an alkylalkoxysilane and an arylalkoxysilane are mentioned. Examples thereof include methyltrimethoxysilane, dimethyldimethoxysilane, phenyltrimethoxysilane, methyltriethoxysilane, dimethyldiethoxysilane, phenyltriethoxysilane, n-propyltrimethoxysilane, n-propyltriethoxysilane, hexyltrimethoxysilane, hexyltriethoxysilane, octyltriethoxysilane, and decyltrimethoxysilane.

Of them, at least one selected from the group consisting of dimethylpolysiloxane, methylhydrogenpolysiloxane, stearic acid, isostearic acid, and octyltriethoxysilane is preferably used as the surface treatment agent, since dispersion in water can be made more satisfactory. Further of these, octyltriethoxysilane is preferably used.

In the hydrophobic treatment for the surface of the inorganic powder, the hydrophobic treatment of a surface is preferably performed at a ratio of 1 to 12 mass% or 2 to 12 mass% relative to the total amount of the inorganic powder after the treatment. When the content is the lower limit (described above) or more, hydrophobicity is sufficiently maintained, whereas when the content is the upper limit (described above) or less, the effect of hydrophobicity is saturated and thus a cost benefit can be enjoyed.

A treatment method for obtaining the inorganic powder with a hydrophobized surface includes dissolving or dispersing a surface treatment agent in an appropriate organic solvent, and stirring the mixed liquid with a predetermined powder, followed by removing the organic solvent. In this manner, a powder with a hydrophobized surface can be obtained.

Examples of the organic solvent used herein include alcohols such as ethanol, isopropyl alcohol and isobutanol; hydrocarbon-based organic solvents such as toluene, n-hexane and cyclohexane; and polar organic solvents such as acetone, ethyl acetate and butyl acetate.

The aqueous dispersion of the invention preferably contains component (A) in a ratio of 10 to 60 mass%, 15 to 55 mass%, or 20 to 50 mass% relative to the total amount of the aqueous dispersion. When the ratio of component (A) falls within the above range, the dispersibility of the aqueous dispersion is further improved.

### (Component (B): Dialkyl Sulfosuccinate and/or Alkyl Sulfoacetate)

The dialkyl sulfosuccinate as component (B) is obtained by reacting, for example, an alcohol with maleic anhydride to form a diester, followed by sulfating the diester. The alcohol to be used is preferably a linear or branched alcohol having 6 to 22 carbon atoms, and more preferably a linear or branched alcohol having 6 to 12 carbon atoms. The alcohol may be a saturated or unsaturated alcohol and is preferably a saturated alcohol. Examples of the alcohol to be used include hexanol, octanol, decanol, lauryl alcohol, myristyl alcohol, cetyl alcohol, stearyl alcohol, oleyl alcohol, 2-ethylhexanol, and 2-methyldodecanol. Of them, 2-ethylhexanol, hexanol, octanol, decanol, or lauryl alcohol is preferable as the alcohol to be used. Examples of the salt of the dialkyl sulfosuccinate include alkali metals such as sodium and potassium; lower alkanolamines such as monoethanolamine, diethanolamine, and triethanolamine; and ammonia. Of them, a salt of an alkali metal such as sodium or potassium, and a sodium salt is preferably used. As the dialkyl sulfosuccinate, a commercially available product can be used.

The two alkyl groups constituting a dialkyl sulfosuccinate are preferably each independently a linear or branched alkyl group having 6 to 22 carbon atoms, and more preferably a linear or branched alkyl group having 6 to 12 carbon atoms. Examples of the alkyl group include a n-hexyl group, a n-octyl group, a 2-ethylhexyl group, a n-decyl group, a lauryl group, a myristyl group, and a stearyl group.

Examples of the dialkyl sulfosuccinate may include dihexylsodium sulfosuccinate, sodium di(2-ethylhexyl) sulfosuccinate, and dilaurylsodium sulfosuccinate. Sodium di(2-ethylhexyl) sulfosuccinate is particularly preferable.

Further, the alkyl sulfoacetate as component (B) is an ester salt of an alcohol (ROH) and sulfoacetic acid (C₂H₄O₅S). Examples of the alkyl sulfoacetate include salts of an alkyl sulfoacetic acid having an alkyl group (R of the alcohol) of 8 to 20 carbon atoms (the number of carbon atoms is preferably 10 to 20, 10 to 18, or 10 to 16). Examples of the alkylsulfoacetate include alkali metals such as sodium and potassium; lower alkanolamines such as monoethanolamine, diethanolamine, and triethanolamine; and ammonia. Of them, a salt of an alkali metal such as sodium or potassium, and a sodium salt is preferably used. As the alkyl sulfoacetate, a commercially available product can be used. The alkyl sulfoacetate is preferably sodium capryl sulfoacetate, sodium lauryl sulfoacetate or sodium myristyl sulfoacetate, and more preferably sodium lauryl sulfoacetate.

To improve dispersibility, component (B) is particularly preferably sodium di(2-ethylhexyl) sulfosuccinate and/or sodium lauryl sulfoacetate.

The compounds as component (B) may be used alone or in combination of two types or more.

The content of component (B) in the aqueous dispersion of the invention is appropriately set. The content may be, for example, 1 mass% or more and 10 mass% or less, preferably 3 mass% or more and 8 mass% or less, 4 mass% or more and 7 mass% or less, or 5 mass% or more and 6 mass% or less in the aqueous dispersion. When the content is 1 mass% or more, the dispersibility of the inorganic powder with a hydrophobized surface in water is further improved, whereas when the content is 10 mass% or less, a cosmetic prepared by adding the water dispersant is further improved in the feeling in use.

### (Component (C): Water)

Component (C) in the aqueous dispersion of the invention is water. Specifically, the water is preferably purified water. For example, deionized water (ion-exchanged water), pure water, ultrapure water, or distilled water is preferably used.

The content of component (C) in the aqueous dispersion of the invention is appropriately set. The content is, for example, 30 to 85 mass% and may be 35 to 80 mass% in the dispersion.

### (Other components)

The aqueous dispersion of the invention preferably does not contain components other than components (A), (B), and (C) in a proportion exceeding 20% by mass in the aqueous dispersion. As long as the performance of the water dispersion is not impaired, the aqueous dispersion may contain other components in a proportion of 20% by mass or less,, more preferably 10 mass% or less, 5 mass% or less, 3 mass% or less, 1 mass% or less, or 0 mass% relative to the aqueous dispersion. However, it is preferable not to add compounds that are likely to impair the stability of the aqueous dispersion.

Examples of the components that may be added include, but are not particularly limited to, a polyhydric alcohol and an antiseptic agent. That is, the dispersion composed of the above components may spoil depending on the conditions and period of storage. In order to prevent such spoilage, a polyhydric alcohol or an antiseptic agent may be added. The polyhydric alcohol and the antiseptic agent are not particularly limited. Examples of the polyhydric alcohol include propylene glycol, 1,3-butylene glycol, dipropylene glycol, pentylene glycol, hexylene glycol, propanediol, and 1,2-hexanediol. The polyhydric alcohols may be used alone, or in combination of two or more types. Examples of the antiseptic agent include para-benzoic acid esters such as methyl paraoxybenzoate, sodium benzoate, phenoxyethanol, and sodium dehydroacetate. The antiseptic agents may be used alone, or in combination of two or more types. These components can be added within the range where each of them exhibits antiseptic performance and has no adverse effect on the performance of the dispersant.

The polyhydric alcohol and the antiseptic agent described above can be added in total in an amount of, for example, 20 mass% or less, or 15 mass% or less, more preferably 10 mass% or less, and may be 5 mass% or less, 3 mass% or less, 1 mass% or less, or 0.5 mass% or less relative to the aqueous dispersion. Of them, a polyhydric alcohol such as propylene glycol, 1,3-butylene glycol, dipropylene glycol, pentylene glycol, hexylene glycol, propanediol, or 1,2-hexanediol is particularly preferably contained in the aqueous dispersion; at least one selected from the group consisting of 1,3-butylene glycol, dipropylene glycol, pentylene glycol, and 1,2-hexanediol is preferably contained; and at least one selected from the group consisting of 1,3-butylene glycol, pentylene glycol, and 1,2-hexanediol is particularly preferably contained. The polyhydric alcohol is often used in cosmetics as a moisturizing agent, however, since the polyhydric alcohol has an antibacterial property, it has an effect as an antiseptic agent.

Furthermore, a component that may be added to the aqueous dispersion include a pH adjuster. Examples of the pH adjuster include buffering agents which buffer between lactic acid and sodium lactate, between citric acid and sodium citrate, and between succinic acid and sodium succinate. The pH adjusters may be used alone, or in combination of two or more types. Examples of the component that may be added to the aqueous dispersion include a sequestering agent. Examples of the sequestering agent include 1-hydroxyethane-1,1-diphosphonic acid, 1-hydroxyethane-1,1-diphosphonate tetrasodium salt, disodium edetate, trisodium edetate, tetrasodium edetate, sodium citrate, sodium polyphosphate, sodium metaphosphate, gluconic acid, phosphoric acid, citric acid, ascorbic acid, succinic acid, edetic acid, and trisodium ethylenediamine hydroxyethyl triacetate. The sequestering agents may be used alone, or in combination of two or more types. The addition amount of the pH adjuster and sequestering agent can be, in total, for example, 1 mass% or less, or 0.5 mass% or less relative to the aqueous dispersion.

A preferred embodiment of the invention is an aqueous dispersion of an inorganic powder with a hydrophobized surface, consisting only of (A) an inorganic powder with a hydrophobized surface, (B) a dialkyl sulfosuccinate and/or an alkyl sulfoacetate, and (C) water.

Another preferred embodiment of the invention is an aqueous dispersion of an inorganic powder with a hydrophobized surface, consisting only of (A) an inorganic powder with a hydrophobized surface, (B) a dialkyl sulfosuccinate and/or an alkyl sulfoacetate, (C) water, and a polyhydric alcohol and/or an antiseptic agent. The polyhydric alcohol is at least one selected from the group consisting of propylene glycol, 1,3-butylene glycol, dipropylene glycol, pentylene glycol, hexylene glycol, propanediol, and 1,2-hexanediol. Furthermore, the polyhydric alcohol is at least one selected from the group consisting of 1,3-butylene glycol, pentylene glycol, and 1,2-hexanediol. The antiseptic agent is at least one selected from the group consisting of para-benzoic acid ester, sodium benzoate, phenoxyethanol, ethanol, and sodium dehydroacetate. The content of the polyhydric alcohol and/or the antiseptic agent in this embodiment may be, in total, 0.1 to 20 mass%, 0.5 to 15 mass%, 1 to 15 mass%, or 2 to 10 mass% in the aqueous dispersion.

Another preferred embodiment of the invention is an aqueous dispersion of an inorganic powder with a hydrophobized surface, consisting only of (A) an inorganic powder with a hydrophobized surface, (B) a dialkyl sulfosuccinate and/or an alkyl sulfoacetate, (C) water, and a polyhydric alcohol. The polyhydric alcohol is at least one selected from the group consisting of propylene glycol, 1,3-butylene glycol, dipropylene glycol, pentylene glycol, hexylene glycol, propanediol, and 1,2-hexanediol. Furthermore, the polyhydric alcohol is at least one selected from the group consisting of 1,3-butylene glycol, pentylene glycol, and 1,2-hexanediol. The content of the polyhydric alcohols in this embodiment may be, in total, 0.1 to 20 mass%, 0.5 to 15 mass%, 1 to 15 mass%, or 2 to 10 mass% in the aqueous dispersion.

Another preferred embodiment of the invention is an aqueous dispersion of an inorganic powder with a hydrophobized surface, consisting only of (A) an inorganic powder with a hydrophobized surface, (B) a dialkyl sulfosuccinate and/or an alkyl sulfoacetate, (C) water, and at least one selected from the group consisting of a polyhydric alcohol, an antiseptic agent, a pH adjuster, and a sequestering agent. The content of at least one selected from the group consisting of a polyhydric alcohol, an antiseptic agent, a pH adjuster, and a sequestering agent in this embodiment may be, in total, 0.1 to 20 mass%, 0.5 to 15 mass%, 1 to 15 mass%, or 2 to 10 mass% in the aqueous dispersion.

The aqueous dispersion of the invention is usually present in a liquid state. Specifically, the aqueous dispersion has a viscosity of, for example, 10 to 5000 mPa·s as measured by a type B viscometer.

The dispersion method for obtaining the aqueous dispersion of the invention may be any method as long as a homogeneous dispersion can be obtained by the method. Various types of dispersers such as a wet bead mill, a homomixer, a dispersion mixer, a kneader, a kneading extruder, and a roll mill can be used. A wet bead mill is more preferable.

### <Cosmetic>

Another embodiment of the invention is a cosmetic containing the aqueous dispersion.

The aqueous dispersion of the invention can be directly blended in a cosmetic. In that case, the dispersion and various components to be used in a cosmetic are mixed to obtain the cosmetic (composition). The amount of the aqueous dispersion to blended in a cosmetic is preferably 2 mass% to 80 mass%.

The cosmetic thus obtained can suitably disperse an aqueous dispersion of an inorganic powder with a hydrophobized surface in an aqueous composition. In this manner, an aqueous composition having stable ultraviolet protecting performance can be obtained. Also in the emulsion, the hydrophobically treated powder can be dispersed in the aqueous phase. A cosmetic having such a specific form is preferable because a user may have a feeling different from a conventional cosmetic having a powder dispersed in an oil phase.

The cosmetic containing the dispersion exhibits high water resistance particularly in a system where an aqueous phase is present as an external phase such as an aqueous cosmetic or an oil-in-water cosmetic. In an aqueous cosmetic and an oil-in-water cosmetic, the aqueous dispersion can be uniformly present in the external phase, in other words, the hydrophobically treated powder is uniformly present in the external phase. Thus, when the cosmetic is applied to the skin, a uniform hydrophobic coating film is easily formed of the hydrophobically treated powder. For the reason, the water resistance is considered to be high. Furthermore, in the case of a composition having an aqueous phase as the external phase, such as an aqueous cosmetic or an oil-in-water cosmetic, a composition except a dispersion is obtained and thereafter the dispersion is added to the aqueous phase as the external phase. In this manner, a cosmetic can be easily obtained.

The cosmetic is not particularly limited. A cosmetic raw material as needed is mixed with the aqueous dispersion of the invention to obtain a UV protection cosmetic such as a sunscreen agent, a base makeup cosmetic such as a foundation, and a point makeup cosmetic such as a lipstick. Of them, in the sunscreen agent, the aqueous dispersant can be particularly suitably used.

The cosmetic may have any form such as an aqueous cosmetic, an oil-in-water cosmetic, or a water-in-oil cosmetic.

In cosmetics, a component (D): a water-soluble polymer and/or an inorganic thickener is preferably contained in addition to the aqueous dispersion. In the form, an aqueous cosmetic or an oil-in-water cosmetic are preferable. Since an oil-in-water cosmetic or an aqueous cosmetic contains an aqueous component as a main component, it is easy to obtain light feeling in use. However, in the case where a water-soluble polymer and an inorganic thickener is blended, these are not compatible with an inorganic powder and aggregation or sedimentation of a powder and the like is likely to occur. When the dispersion is used in a system containing a water-soluble polymer and/or an inorganic thickener, it is possible to obtain a cosmetic having no or little aggregation or sedimentation of a powder and excellent in storage stability over time.

As the water-soluble polymer and/or the inorganic thickener as component (D) according to the invention, a commonly known water-soluble polymer and/or an inorganic thickener used in cosmetics can be blended. Examples of the water-soluble polymer include natural polymers such as gum Arabic, tragacanth gum, galactan, guar gum, carob gum, karaya gum, carrageenan, pectin, agar, quince seed (quince), algae colloid (brown alga extract), starch (rice, corn, potato, wheat), glycyrrhizin, xanthan gum, dextran, succinoglucan, pullulan, gellan gum, KERCOGEL, mannan, curdlan, locust bean gum, and sclerotium gum; animal-based polymers such as collagen, casein, albumin, and gelatin; starch-based polymers such as carboxymethyl starch, and methyl hydroxypropyl starch; cellulose based polymers such as methylcellulose, nitrocellulose, ethylcellulose, methylhydroxypropylcellulose, hydroxycellulose, hydroxyethylcellulose, hydroxymethylcellulose, hydroxypropylcellulose, hydroxypropylmethylcellulose, hydrophobized hydroxypropylmethylcellulose, carboxymethylcellulose, sodium carboxymethylcellulose, sodium cellulose sulfate, crystalline cellulose, cellulose gum, cationic cellulose, and stearoxyhydroxypropylmethylcellulose; alginate-based polymers such as alginic acid, sodium alginate, and propylene glycol alginate; vinyl-based polymers such as polyvinyl alcohol, polyvinyl acetate, a copolymer of vinylpyrrolidone and vinyl acetate, polyvinyl methyl ether, polyvinylpyrrolidone, an alkyl-modified carboxyvinyl polymer, and carboxyvinyl polymer (carbomer); polyoxyethylene or polyoxypropylene copolymer-based polymers, such as polyethylene glycol, polyethyleneimine, and (PEG-240/decyltetradeceth-20/HDI) copolymer; and acrylic-based polymers such as sodium polyacrylate, polyacrylamide, polyacrylic alkanolamine, a copolymer of an alkyl methacrylate and dimethylaminoethyl methacrylate, poly 2-acrylamido-2-methylpropanesulfonic acid, polymethacryloyloxytrimethylammonium, an (ammonium acryloyldimethyl taurate/VP) copolymer, a dimethylacrylamide/sodium acryloyldimethyl taurate) cross polymer, a (dimethylacrylamide/sodium acryloyldimethyl taurate) cross polymer, a (sodium acrylate/acryloyldimethyl taurate) copolymer, an (alkyl acrylate/steareth-20 methacrylate) copolymer, an acrylates/alkyl(C10-30) acrylate cross polymer, a (hydroxyethyl acrylate/sodium acryloyldimethyl taurate) copolymer, a (sodium acrylate/sodium acryloyldimethyl taurate) copolymer, an ammonium acryloyldimethyl taurate/beheneth-25 methacrylate cross polymer, polyethyl acrylate, a polyalkylacrylamide/polyacrylamide copolymer, an (ammonium acryloyldimethyl taurate/carboxyethyl acrylate) cross polymer, an (ammonium acryloyldimethyl taurate/VP) copolymer, and polyacrylate cross polymer-6.

Furthermore, examples of the inorganic thickener as component (D) of the invention include anhydrous silicic acid, Al silicate, Na silicate, AlMg silicate, silicic acid (Na/Mg), fluorosilicic acid (Na/Mg), laponite, hectorite, smectite, and bentonite.

Component (D) is preferably at least one selected from the group consisting of xanthan gum, hydroxyethyl cellulose, a carboxyvinyl polymer (carbomer), a (sodium acrylate/sodium acryloyldimethyltaurate) copolymer, polyacrylate cross polymer-6, and fluorosilicic acid (Na/Mg).

The content of component (D) in the cosmetic of the invention is preferably 0.02 to 2 mass%. As long as the content of component (D) falls within this range, the cosmetic becomes stable, and the viscosity tends to be suitable.

The cosmetic may be used in combination with any aqueous component or oily component that can be used in the cosmetic field. Examples of the aqueous component and oily component are not particularly limited to, and components such as an oil, a surfactant, a humectant, a higher alcohol, a sequestering agent, an oil-soluble polymer, an ultraviolet ray filter, other chemical agent components, various extraction liquids, a coloring agent such as an organic dye, an antiseptic, an antioxidant, a dye, a thickener, a pH adjuster, a fragrance, a cooling feeling agent, an antiperspirant, a disinfectant, a skin stimulating agent, and various powders may be included.

Examples of the oil include, but are not particularly limited to, natural animal and vegetable oils (e.g., olive oil, mink oil, castor oil, palm oil, beef tallow, evening primrose oil, coconut oil, castor oil, cocoa oil, macadamia nut oil); waxes (e.g., jojoba oil, beeswax, lanolin, carnauba wax, candelilla wax); higher alcohols (e.g., lauryl alcohol, stearyl alcohol, cetyl alcohol, oleyl alcohol); higher fatty acids (e.g., lauric acid, palmitic acid, stearic acid, oleic acid, behenic acid, lanolin fatty acid); higher aliphatic hydrocarbons (e.g., liquid paraffin, solid paraffin, squalane, petrolatum, ceresin, microcrystalline wax); synthetic ester oils (e.g., butyl stearate, hexyl laurate, diisopropyl adipate, diisopropyl sebacate, octyldodecyl myristate, isopropyl myristate, isopropyl palmitate isopropyl myristate, cetyl isooctanoate, neopentyl glycol dicaprate); and silicone derivatives (e.g., silicone oils such as methyl silicone and methyl phenyl silicone). Furthermore, e.g., an oil-soluble vitamin, an antiseptic agent and a whitening agent can be blended.

Examples of the surfactant include a lipophilic nonionic surfactant and a hydrophilic nonionic surfactant. Examples of the lipophilic nonionic surfactant include, but are not particularly limited to, sorbitan fatty acid esters such as sorbitan monooleate, sorbitan monoisostearate, sorbitan monolaurate, sorbitan monopalmitate, sorbitan monostearate, sorbitan sesquioleate, sorbitan trioleate, diglycerol sorbitan penta-2-ethylhexylate, and diglycerol sorbitan tetra-2-ethylhexylate; glycerol polyglycerol fatty acids such as an ester of glycerin and mono cotton-seed oil fatty acid, glyceryl monoerucate, glyceryl sesquioleate, glyceryl monostearate, α,α'-glyceryl oleate pyroglutamate, and glyceryl monostearate malate; propylene glycol fatty acid esters such as propylene glycol monostearate; hydrogenated castor oil derivatives; and glycerin alkyl ethers.

Examples of the hydrophilic nonionic surfactant include, but are not particularly limited to, POE sorbitan fatty acid esters such as POE sorbitan monooleate, POE sorbitan monostearate, and POE sorbitan tetraoleate; POE sorbitol fatty acid esters such as POE sorbitol monolaurate, POE sorbitol monooleate, POE sorbitol pentaoleate, and POE sorbitol monostearate; POE glycerin fatty acid esters such as POE glycerin monostearate, POE glycerin monoisostearate, and POE glycerin triisostearate; POE fatty acid esters such as POE monooleate, POE distearate, POE monodioleate, and ethylene glycol distearate; POE alkyl ethers such as POE lauryl ether, POE oleyl ether, POE stearyl ether, POE behenyl ether, POE 2-octyldodecyl ether, and POE cholestanol ether; POE alkyl phenyl ethers such as POE octyl phenyl ether, POE nonyl phenyl ether, and POE dinonyl phenyl ether; pluronic types such as Pluronic; POE·POP alkyl ethers such as POE·POP cetyl ether, POE·POP 2-decyl tetradecyl ether, POE·POP monobutyl ether, POE·POP hydrogenated lanolin, and POE·POP glycerin ether; tetra POE·tetra POP ethylenediamine condensates such as tetronic; POE castor oil hydrogenated castor oil derivatives such as POE castor oil, POE hydrogenated castor oil, POE hydrogenated castor oil monoisostearate, POE hydrogenated castor oil triisostearate, POE hydrogenated castor oil monopyroglutamic acid monoisostearate diester, and POE hydrogenated castor oil maleic acid; POE beeswax·lanolin derivatives such as POE sorbitol beeswax; alkanolamides such as a coconut oil fatty acid diethanolamide, a lauric acid monoethanolamide, and a fatty acid isopropanolamide; and POE propylene glycol fatty acid ester, a POE alkylamine, a POE fatty acid amide, a sucrose fatty acid ester, a POE nonylphenyl formaldehyde condensate, an alkylethoxydimethylamine oxide, and trioleyl phosphate.

Examples of the other surfactants include anionic surfactants such as fatty acid soaps, higher alkyl sulfates, POE triethanolamine lauryl sulfates, and alkyl ether sulfates; cationic surfactants such as alkyl trimethyl ammonium salts, alkyl pyridinium salts, alkyl quaternary ammonium salts, alkyl dimethyl benzyl ammonium salts, POE alkyl amines, alkyl amine salts, and polyamine fatty acid derivatives; and amphoteric surfactants such as imidazoline-based amphoteric surfactants and betaine-based surfactants. These may be blended within a range that they do not cause problems in stability and skin irritation.

Examples of the humectant include, but are not particularly limited to, xylitol, sorbitol, maltitol, chondroitin sulfate, hyaluronic acid, mucoitinsulfuric acid, charonin acid, atelocollagen, cholesteryl-12-hydroxystearate, sodium lactate, bile acid salt, dl-pyrrolidone carboxylate, short-chain soluble collagen, diglycerin(EO)PO adduct, Rosa roxburghii extract, Achillea millefolium extract, and melilot extract.

Examples of the higher alcohol include, but are not particularly limited to, linear alcohols such as lauryl alcohol, cetyl alcohol, stearyl alcohol, behenyl alcohol, myristyl alcohol, oleyl alcohol, and cetostearyl alcohol (cetearyl alcohol); and branched alcohols such as monostearyl glycerin ether (batyl alcohol), 2-decyltetradecinol, lanolin alcohol, cholesterol, phytosterol, hexyldodecanol, isostearyl alcohol, and octyldodecanol.

Examples of the sequestering agent include, but are not particularly limited to, 1-hydroxyethane-1,1-diphosphonic acid, tetrasodium 1-hydroxyethane-1,1-diphosphonate, sodium citrate, sodium polyphosphate, sodium metaphosphate, gluconic acid, phosphoric acid, citric acid, ascorbic acid, succinic acid, and edetic acid.

Examples of the ultraviolet ray filter include, but are not particularly limited to, benzoic acid-based ultraviolet ray blockers such as para-aminobenzoic acid (hereinafter abbreviated as PABA), PABA monoglycerin ester, N,N-dipropoxy PABA ethyl ester, N,N-diethoxy PABA ethyl ester, N,N-dimethyl PABA ethyl ester, and N,N-dimethyl PABA butyl ester; anthranilic acid-based ultraviolet ray blockers such as homomenthyl-N-acetylanthranilate; salicylic acid-based ultraviolet ray blockers such as amyl salicylate, menthyl salicylate, homomenthyl salicylate, octyl salicylate, phenyl salicylate, benzyl salicylate, and p-isopropanol phenyl salicylate; cinnamic acid-based ultraviolet ray blockers such as ethylhexyl methoxycinnamate, octyl cinnamate, ethyl-4-isopropyl cinnamate, methyl 2,5-diisopropyl cinnamate, ethyl-2,4-diisopropyl cinnamate, methyl-2,4-diisopropyl cinnamate, propyl-p-methoxycinnamate, isopropyl-p-methoxycinnamate, isoamyl-p-methoxycinnamate, 2-ethoxyethyl-p-methoxycinnamate, cyclohexyl-p-methoxycinnamate, ethyl-α-cyano-β-phenyl cinnamate, 2-ethylhexyl α-cyano-β-phenyl cinnamate, and glyceryl mono-2-ethylhexanoyl-di-para-methoxycinnamate; benzophenone ultraviolet ray blockers such as 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone, 2-hydroxy-4-methoxy-4'-methylbenzophenone, 2-hydroxy-4-methoxybenzophenone-5-sulfonate, 4-phenylbenzophenone, 2-ethylhexyl-4'-phenyl-benzophenone-2-carboxylate, 2-hydroxy-4-n-octoxybenzophenone, and 4-hydroxy-3-carboxybenzophenone; and 3-(4'-methylbenzylidene)-d,l-camphor, 3-benzylidene-d,l-camphor, urocanic acid, urocanic acid ethyl ester, 2-phenyl-5-methylbenzoxazole, 2,2'-hydroxy-5-methylphenylbenzotriazole, 2-(2'-hydroxy-5'-t-octylphenyl)benzotriazole, 2-(2'-hydroxy-5'-methylphenylbenzotriazole, dibenzalazine, dianisoylmethane, 4-methoxy-4'-t-butyldibenzoylmethane, and 5-(3,3-dimethyl-2-norbornylidene)-3-pentan-2-one.

Examples of the other chemical agent components include, but are not particularly limited to, vitamins such as vitamin A oil, retinol, retinol palmitate, inositol, pyridoxine hydrochloride, benzyl nicotinate, nicotinamide, DL-α-tocopherol nicotinate, magnesium ascorbyl phosphate, 2-O-α-D-glucopyranosyl-L-ascorbic acid, vitamin D2 (ergocaciferol), dl-α-tocopherol, dl-α-tocopherol acetate, pantothenic acid, and biotin; hormones such as estradiol and ethinylestradiol; amino acids such as arginine, aspartic acid, cystine, cysteine, methionine, serine, leucine, and tryptophan; anti-inflammatory agents such as allantoin and azulene; skin lightening agents such as arbutin; astringents such as tannic acid; cooling agents such as L-menthol and camphor, sulfur, lysozyme chloride, and pyridoxine chloride.

Examples of the extraction liquids include, but are not particularly limited to, Houttuynia cordata extract, Phellodendron bark extract, Melilot extract, Lamium extract, Licorice extract, Peony extract, soapwort extract, loofah extract, cinchona extract, saxifrage extract, Sophora flavescens extract, water hyacinth extract, Foeniculum vulgare extract, Primula Veris Flower Extract, rose extract, Rehmannia root extract, lemon extract, Lithospermum root extract, aloe extract, calamus root extract, eucalyptus extract, horsetail extract, sage extract, thyme extract, tea extract, seaweed extract, cucumber extract, clove extract, Rubus idaeus extract, Hamamelis x intermedia extract, carrot extract, horse chestnut extract, peach extract, peach leaf extract, mulberry extract, cornflower extract, hamamelis extract, placenta extract, thymus extract, silk extract, and licorice extract.

Examples of the various powders include bright color pigments such as red iron oxide, yellow iron oxide, black iron oxide, mica titanium, iron oxide-coated mica titanium, and titanium oxide-coated glass flakes; inorganic powders such as mica, talc, kaolin, and sericite; and organic powders such as polyethylene powder, nylon powder, crosslinked polystyrene, cellulose powder, and silicone powder. Preferably, in order to improve the sensory characteristics and the lasting of makeup, a part or all of the powder components are hydrophobized with a substance such as a silicone, a fluorine compound, a metal soap, an oil, or an acyl glutamate, in accordance with a commonly known method and put in use. Alternatively, a composite powder that does not correspond to the inorganic powder with a hydrophobized surface may be mixed and put in use.

### Examples

The invention will be described in more detail based on Examples, but the invention is not limited to these Examples alone. In Examples and Comparative Examples, "%" means mass% unless otherwise specified.

### (Examples 1 and 2, Comparative Examples 1 to 12)

In a mayonnaise bottle, 75 g of water (purified water) and 5 g of component (B) in Table 1 (or component (B') corresponding to component (B)) were added and further 20 g of a powder obtained by treating the surface of fine titanium oxide particles (MT-05: particle diameter 10 nm, manufactured by TAYCA CORPORATION) with octyltriethoxysilane (5 mass%) and 100 g of ϕ1.5 mm glass beads were added. After the mixture was dispersed by a paint shaker, the beads were separated to obtain aqueous dispersions of Examples 1 and 2 and Comparative Examples 1 to 12 as shown in Tables 1 and 2.

### (Example 3)

In a mayonnaise bottle, 75 g of water (purified water) and 5 g of sodium di(2-ethylhexyl) sulfosuccinate (NIKKOL OTP-100, manufactured by Nikko Chemicals) were added, and further 20 g of a powder obtained by treating the surface of fine titanium oxide particles (MT-100SA: particle diameter 15 nm, manufactured by TAYCA CORPORATION) with methyl hydrogen polysiloxane (5 mass%) and 100 g of ϕ1.5 mm glass beads were added. After the mixture was dispersed by a paint shaker, the beads were separated to obtain a homogeneous aqueous dispersion.

### (Example 4)

In a mayonnaise bottle, 75 g of water (purified water) and 5 g of sodium di(2-ethylhexyl) sulfosuccinate (NIKKOL OTP-100, manufactured by Nikko Chemicals) were added, and further 20 g of a powder obtained by treating the surface of fine titanium oxide particles (MT-05: particle diameter 10 nm, manufactured by TAYCA CORPORATION) with stearic acid (5 mass%) and 100 g of ϕ1.5 mm glass beads were further added. After the mixture was dispersed by a paint shaker, the beads were separated to obtain a homogeneous aqueous dispersion.

### (Example 5)

In a mayonnaise bottle, 75 g of water (purified water) and 5 g of sodium lauryl sulfoacetate (NIKKOL LSA-F, manufactured by Nikko Chemicals) were added and further 20 g of a powder obtained by treating the surface of fine titanium oxide particles (MT-100SA: particle diameter 15 nm, manufactured by TAYCA CORPORATION) with isostearic acid (5 mass%) and 100 g of ϕ1.5 mm glass beads were added. After the mixture was dispersed by a paint shaker, the beads were separated to obtain a homogeneous aqueous dispersion.

### (Example 6)

In a mayonnaise bottle, 41 g of water (purified water), 5 g of sodium di(2-ethylhexyl) sulfosuccinate (NIKKOL OTP-100, manufactured by Nikko Chemicals), and 4 g of 1,3-butylene glycol were added, and further 50 g of powder obtained by treating the surface of fine zinc oxide particles (MZ-500: particle diameter 25 nm, manufactured by TAYCA CORPORATION) with octyltriethoxysilane (8 mass%) and 100 g of ϕ1.5 mm glass beads were added. After the mixture was dispersed by a paint shaker, the beads were separated to obtain a homogeneous aqueous dispersion.

### (Example 7)

In a mayonnaise bottle, 51 g of water (purified water), 6 g of sodium di(2-ethylhexyl) sulfosuccinate (NIKKOL OTP-100, manufactured by Nikko Chemicals) sulfosuccinate, and 3 g of dipropylene glycol were added, and further 40 g of a powder obtained by treating the surface of fine titanium oxide particles (MT-05: particle diameter 10 nm, manufactured by TAYCA CORPORATION) with octyltriethoxysilane (5 mass%) and 100 g of ϕ1.5 mm glass beads were added. After the mixture was dispersed by a paint shaker, the beads were separated to obtain a homogeneous aqueous dispersion.

### (Example 8)

In a mayonnaise bottle, 51 g of water (purified water), 6 g of sodium di(2-ethylhexyl) sulfosuccinate (NIKKOL OTP-100, manufactured by Nikko Chemicals), and 3 g of pentylene glycol were added, and further 40 g of yellow iron oxide obtained by treating the surface thereof with octyltriethoxysilane (2 mass%) (OTS-2 YELLOW LL-100P, manufactured by Daito Kasei Co., Ltd.) and 100 g of ϕ1.5 mm glass beads were added. After the mixture was dispersed by a paint shaker, the beads were separated to obtain a homogeneous aqueous dispersion.

### (Example 9)

In a mayonnaise bottle, 75 g of water (purified water) and 5 g of sodium di(2-ethylhexyl) sulfosuccinate (NIKKOL OTP-100, manufactured by Nikko Chemicals) were added, and further 20 g of fine particle cerium oxide obtained by treating the surface thereof with methyl hydrogen polysiloxane (5 mass%) (SI01-5 Serigard SC6832: particle diameter 35 nm, manufactured by Daito Kasei Co., Ltd.) and 100 g of ϕ1.5 mm glass beads were added. After the mixture was dispersed by a paint shaker, the beads were separated to obtain a homogeneous aqueous dispersion.

The dispersion states of the aqueous dispersions obtained in Examples 1 to 9 and Comparative Examples 1 to 12 immediately after production and after storage at 45°C for 1 month were visually observed.

The state where powder is homogenously dispersed was evaluated as "∘"; the state where powder is well dispersed in water but rich in foams and lumps was evaluated as "△"; and the state where water and most of the powder were separated was evaluated as "×". The results are shown in Tables 1 and 2.

**[Table 1]**

| | Component B | | Inorganic powder with hydrophobized surface | Concentration of powder (%) | Dispersion state | |
|---|---|---|---|---|---|---|
| | | | | | Immediately after production | After one-month storage |
| Example 1 | Sodium di(2-ethylhexyl) sulfosuccinate | NIKKOL OTP-100 | Fine titanium oxide particles treated with octyltriethoxysilane | 20 | ○ | ○ |
| Example 2 | Sodium laurylsulfoacetate | NIKKOL LSA-F | Fine titanium oxide particles treated with octyltriethoxysilane | 20 | ○ | ○ |
| Example 3 | Sodium di(2-ethylhexyl) sulfosuccinate | NIKKOL OTP-100 | Fine titanium oxide particles treated with methylhydrogenpolysiloxane | 20 | ○ | ○ |
| Example 4 | Sodium di(2-ethylhexyl) sulfosuccinate | NIKKOL OTP-100 | Fine titanium oxide particles treated with stearic acid | 20 | ○ | ○ |
| Example 5 | Sodium laurylsulfoacetate | NIKKOL LSA-F | Fine titanium oxide particles treated with isostearic acid, | 20 | ○ | ○ |
| Example 6 | Sodium di(2-ethylhexyl) sulfosuccinate | NIKKOL OTP-100 | Fine zinc oxide particles treated with octyltriethoxysilane | 50 | ○ | ○ |
| Example 7 | Sodium di(2-ethylhexyl) sulfosuccinate | NIKKOL OTP-100 | Fine titanium oxide particles treated with octyltriethoxysilane | 40 | ○ | ○ |
| Example 8 | Sodium di(2-ethylhexyl) sulfosuccinate | NIKKOL OTP-100 | Yellow iron oxide treated with octyltriethoxysilane | 40 | ○ | ○ |
| Example 9 | Sodium di(2-ethylhexyl) sulfosuccinate | NIKKOL OTP-100 | Fine particle cerium oxide particles treated with methylhydrogenpolysiloxane | 20 | ○ | ○ |

| | | | | | | |
|---|---|---|---|---|---|---|
| Note that NIKKOL in the table is manufactured by Nikko Chemicals | | | | | | |

**[Table 2]**

| | Component B' | | Inorganic powder with hydrophobized surface | Concentration of powder (%) | Dispersion state | |
|---|---|---|---|---|---|---|
| | | | | | Immediately after production | After one-month storage |
| Comparative Example 1 | Polyoxyethylene behenyl ether | NIKKOL BB-30 | Fine titanium oxide particles treated with octyltriethoxysilane | 20 | × | - |
| Comparative Example 2 | Polyoxyethylene cetyl ether | NIKKOL BC-25 | Fine titanium oxide particles treated with octyltriethoxysilane | 20 | × | - |
| Comparative Example 3 | Sodium polyoxyethylene lauryl ether phosphate | NIKKOL DLP-10 | Fine titanium oxide particles treated with octyltriethoxysilane | 20 | Δ | × |
| Comparative Example 4 | Sodium lauroyl sarcosinate | NIKKOL sarcosinate LN | Fine titanium oxide particles treated with octyltriethoxysilane | 20 | × | - |
| Comparative Example 5 | Potassium laurate | NIKKOL potassium laurate LK-120 | Fine titanium oxide particles treated with octyltriethoxysilane | 20 | × | - |
| Comparative Example 6 | Sodium lauryl phosphate | NIKKOL SLP-N | Fine titanium oxide particles treated with octyltriethoxysilane | 20 | × | - |
| Comparative Example 7 | Sodium lauroylmethyltaurate | NIKKOL LMT | Fine titanium oxide particles treated with octyltriethoxysilane | 20 | × | - |
| Comparative Example 8 | Sodium lauryl sulfate | NIKKOL SLS | Fine titanium oxide particles treated with octyltriethoxysilane | 20 | × | - |
| Comparative Example 9 | Sodium polyoxyethylene laurylether acetate | NIKKOL AKYPO RLM 45N | Fine titanium oxide particles treated with octyltriethoxysilane | 20 | × | - |
| Comparative | Sodium | PELICERr L-30 | Fine titanium oxide | 20 | × | - |
| Example 10 | dilauramidoglutamide lysine | | particles treated with octyltriethoxysilane | | | |
| Comparative Example 11 | PEG-10 dimethicone | KF-6043 | Fine titanium oxide particles treated with octyltriethoxysilane | 20 | Δ | Δ |
| Comparative Example 12 | Polyglyceryl monolaurate | NIKKOL Decaglyn 1-L | Fine titanium oxide particles treated with octyltriethoxysilane | 20 | Δ | Δ |

In the table, NIKKOL is manufactured by Nikko Chemicals, PELICER L-30 is manufactured by Asahi Kasei Finechem Corporation, and KF-6043 is manufactured by Shin-Etsu Chemical Co., Ltd.

From the results shown in Tables 1 and 2, it is apparent that the aqueous dispersions of the invention have a good dispersion state and can be suitably used for cosmetics. The aqueous dispersions of Examples 1 to 9 had a contact angle of 90 to 95° in a water repellency test described later, and excellent water resistance.

### (Example 10: Oil-in-Water Sunscreen Cream)

### (Components)

| | |
|---|---|
| 1. Polyacrylic acid | emulsifier (containing water-soluble polymer) (note 1) 1.5 (%) |
| 2. 1,3-Butylene glycol | 3.0 |
| 3. Antiseptic agent | 0.1 |
| 4. Purified Water | 62.4 |
| 5. Ethylhexyl methoxycinnamate | 7.5 |
| 6. Dextrin palmitate | 0.5 |
| 7. Decamethylcyclopentasiloxane | 5.0 |
| 8. Crosslinked dimethylpolysiloxane | 5.0 |
| 9. Aqueous dispersion of Example 6 | 5.0 |
| 10. Aqueous Dispersion of Example 7 | 10.0 |

| | |
|---|---|
| (note 1): Polyacrylic acid-based emulsifier; Simargel EG (manufactured by SEPPIC S.A.) ((sodium acrylate/sodium acryloyldimethyltaurine) copolymer, isohexadecane, polysorbate 80, water; (sodium acrylate/acryloyldimethyltaurine) copolymer is contained in an amount of 35 to 40 mass%) | |

### (Production Method)

A: Components 1 to 4 are mixed and homogenized.
B: Components 5 and 6 are mixed and heated to be homogenous.
C: The components 7 and 8 are mixed and homogenized.
D: B and C are added to A and emulsified.
E: Components 9 and 10 were added to D to obtain an oil-in-water sunscreen cream.

The oil-in-water sunscreen cream obtained as described above spread easily, provided a fresh and refreshing feeling of use. Furthermore water resistance (contact angle in the water repellency test described later was 85°) was high, makeup satisfactorily lasted long, and stability was excellent without a change over time.

Note that the feeling of use (freshness) was evaluated by 10 trained expert panelists of 25 to 40 years old based on the following criteria of feeling of use (freshness). The result was good (∘)
∘; 7 or more out of the 10 monitors evaluated as good.
△; 4 to 6 out of the 10 monitors evaluated as good.
×; 0 to 3 of the 10 monitors evaluated as good.

### (Example 11: Water-in-Oil Sunscreen Cream)

### (Components)

| | |
|---|---|
| 1. Ethylhexyl methoxycinnamate | 5.0 (%) |
| 2. Hexyl diethylaminohydroxybenzoylbenzoate | 1.0 |
| 3. Isotridecyl isononanoate | 6.0 |
| 4. Decamethylcyclopentasiloxane | 7.0 |
| 5. Alkyl/polyether co-modified silicone (note 1) | 1.5 |
| 6. Aqueous Dispersion of Example 7 | 5.0 |
| 7. 1,3-Butylene glycol | 5.0 |
| 8. Ethanol | 8.0 |
| 9. Sodium Citrate | 0.2 |
| 10. Magnesium sulfate | 0.5 |
| 11. Purified Water | 60.8 |

| | |
|---|---|
| (note 1) Alkyl/polyether co-modified silicone; KF-6038 (manufactured by Shin-Etsu Chemical Co., Ltd.) | |

### (Production Method)

A: Components 1 to 5 were mixed and homogenized.
B: Components 6 to 11 were mixed and dissolved to be homogeneous.
C: B was added to A and emulsified to obtain a water-in-oil sunscreen cream.

The water-in-oil sunscreen cream obtained as described above had fine texture, satisfactory spreadability without stickiness or greasiness. Furthermore, the cream was moist and fresh, and provides a fresh feeling in use as well as makeup satisfactorily lasted long. Moreover, water resistance thereof (contact angle in the water repellency test described later was 110°) was high, and stability was excellent without a change over time.

### (Example 12: Oil-in-Water Emulsion Foundation)

### (Components)

| | |
|---|---|
| 1. Decamethylcyclopentasiloxane | 20.0 (%) |
| 2. Ethylhexyl methoxycinnamate | 3.0 |
| 3. Dimethylpolysiloxane (10 cs) | 5.0 |
| 4. Silicone treated titanium oxide | 8.0 |
| 5. Silicone treated red iron oxide | 0.4 |
| 6. Silicone treated yellow iron oxide | 1.0 |
| 7. Silicone-treated black iron oxide | 0.1 |
| 8. Stearoxyhydroxypropyl methylcellulose (note 1) | 0.3 |
| 9. Antiseptic agent | 0.1 |
| 10. Purified Water | 49.1 |
| 11. 1,3-Butylene glycol | 3.0 |
| 12. Aqueous Dispersion of Example 4 | 10.0 |

| | |
|---|---|
| (note 1) Stearoxyhydroxypropyl methylcellulose; SANGELOSE 90 L (manufactured by Daido Chemical Corporation) | |

### (Production Method)

A: Components 8 to 11 were mixed and dissolved by heating.
B: Components 1 to 3 were mixed, components 4 to 7 were added thereto, and dispersed and mixed with a dispersion mixer.
C: B was added to A and emulsified, and component 12 was added to obtain oil-in-water emulsion foundation.

The oil-in-water type emulsion foundation obtained as described above was excellent in feeling in use (evaluation ∘) such as satisfactory spreadability in use, no stickiness, and freshness. Furthermore, makeup lasted extremely long. Moreover water resistance thereof was high (contact angle in water repellency test described later: 90°) and storage stability was satisfactory.

### (Example 13: Water-in-Oil Emulsion Foundation)

### (Components)

| | |
|---|---|
| 1. Decamethylcyclopentasiloxane | 16.6 (%) |
| 2. Isotridecyl isononanoate | 4.0 |
| 3. Ethylhexyl methoxycinnamate | 4.0 |
| 4. Polyether-modified silicone (note 1) | 2.0 |
| 5. Crosslinked dimethylpolysiloxane | 1.0 |
| 6. Silicone treated titanium oxide | 8.0 |
| 7. Silicone treated red iron oxide | 0.4 |
| 8. Silicone treated yellow iron oxide | 1.0 |
| 9. Silicone-treated black iron oxide | 0.1 |
| 10. POE sorbitan monooleate (20EO) | 0.3 |
| 11. 1,3-Butylene glycol | 4.0 |
| 12. Dispersion of Example 3 | 5.0 |
| 13. Glycerin | 4.0 |
| 14. Magnesium sulfate | 1.0 |
| 15. Ethanol | 5.0 |
| 16. Antiseptic agent | 0.1 |
| 17. Purified water | 43.5 |

| | |
|---|---|
| (note 1) Polyether-modified silicone; ES-5612 (manufactured by Dow Corning Toray Co., Ltd.) | |

### (Production Method)

A: Components 1 to 5 were mixed and homogenized.
B: 6 to 9 were added to the components 10 and 11 and dispersed by a roller.
C: B and components 12 to 17 were mixed and homogenized.
D: C was added to A and emulsified to obtain water-in-oil emulsion foundation.

The water-in-oil emulsion foundation obtained as described above was excellent in feeling in use (evaluation ∘) such as satisfactory spreadability in use, no stickiness, and freshness. Furthermore, makeup lasted extremely long. Moreover water resistance thereof was high (contact angle in water repellency test described later: 105°) and storage stability was satisfactory.

### (Example 14)

In a mayonnaise bottle, 45 g of purified water and 5 g of sodium di(2-ethylhexyl) sulfosuccinate (NIKKOL OTP-100, manufactured by Nikko Chemicals) were added, and further 50 g of a powder obtained by treating the surface of fine titanium oxide particles (MT-05: particle diameter 10 nm, manufactured by TAYCA CORPORATION) with octyltriethoxysilane (5 mass%) and 100 g of ϕ1.5 mm glass beads were added. After the mixture was dispersed by a paint shaker and the beads were separated, a homogeneous aqueous dispersion having a powder concentration of 50% was obtained.

### (Example 15)

In a mayonnaise bottle, 41 g of purified water, 5 g of sodium lauryl sulfoacetate (NIKKOL LSA-F, manufactured by Nikko Chemicals), and 4 g of 1,3-butylene glycol were added, and further 50 g of powder obtained by treating the surface of fine zinc oxide particles (MZ-500: particle diameter 25 nm, manufactured by TAYCA CORPORATION) with octyltriethoxysilane (8 mass%) and 100 g of ϕ1.5 mm glass beads were added. After the mixture was dispersed by a paint shaker and the beads were separated, a homogeneous aqueous dispersion having a powder concentration of 50% was obtained.

### (Example 16)

In a mayonnaise bottle, 45 g of purified water, 5 g of sodium di(2-ethylhexyl) sulfosuccinate (NIKKOL OTP-100, manufactured by Nikko Chemicals), and 3 g of pentylene glycol were added, and further 50 g of pigment-grade titanium oxide obtained by treating the surface thereof with octyltriethoxysilane (2 mass%) (OTS-2 CR-50, particle diameter 0.25 µm, manufactured by Daito Kasei Co., Ltd.) and 100 g of ϕ1.5 mm glass beads were added. After the mixture was dispersed by a paint shaker and the beads were separated, a homogeneous aqueous dispersion having a powder concentration of 50% was obtained.

### (Example 17)

In a mayonnaise bottle, 45 g of purified water, 5 g of sodium di(2-ethylhexyl) sulfosuccinate (NIKKOL OTP-100, manufactured by Nikko Chemicals), and 3 g of pentylene glycol were added, and further 50 g of red iron oxide obtained by treating the surface thereof with octyltriethoxysilane (2 mass%) (OTS-2 R-516P, particle diameter 0.5 µm, manufactured by Daito Kasei Co., Ltd.) and 100 g of ϕ1.5 mm glass beads were added. After the mixture was dispersed by a paint shaker and the beads were separated, a homogeneous aqueous dispersion having a powder concentration of 50% was obtained.

### (Example 18)

In a mayonnaise bottle, 45 g of purified water, 5 g of sodium di(2-ethylhexyl) sulfosuccinate (NIKKOL OTP-100, manufactured by Nikko Chemicals), and 3 g of pentylene glycol were added, and further 50 g of yellow iron oxide obtained by treating the surface thereof with octyltriethoxysilane (2 mass%) (OTS-2 YELLOW LL-100P, particle diameter 0.7 µm, manufactured by Daito Kasei Co., Ltd.) and 100 g of ϕ1.5 mm glass beads were added. After the mixture was dispersed by a paint shaker and the beads were separated, a homogeneous aqueous dispersion having a powder concentration of 50% was obtained.

### (Example 19)

In a mayonnaise bottle, 45 g of purified water, 5 g of sodium di(2-ethylhexyl) sulfosuccinate (NIKKOL OTP-100, manufactured by Nikko Chemicals), and 3 g of pentylene glycol were added, and further 50 g of black iron oxide obtained by treating the surface thereof with octyltriethoxysilane (2 mass%) (OTS-2 BL-100P, particle diameter 0.2 µm, manufactured by Daito Kasei Co., Ltd.) and 100 g of ϕ1.5 mm glass beads were added. After the mixture was dispersed by a paint shaker and the beads were separated, a homogeneous aqueous dispersion having a powder concentration of 50% was obtained.

### (Comparative Production Example 13)

In a mayonnaise bottle, 26 g of purified water, 12 g of PEG-10 dimethicone (KF-6043, manufactured by Shin-Etsu Chemical Co., Ltd.), and 12 g of 1,3-butylene glycol were added, and further 50 g of a powder fine titanium oxide particles obtained by treating the surface thereof with hydrogen dimethicone (MTY-100SAS: particle diameter 15 nm, manufactured by TAYCA CORPORATION) and 100 g of ϕ1.5 mm glass beads were further added. After the mixture was dispersed by a paint shaker and the beads were separated, an aqueous dispersion having a powder concentration of 50% was obtained.

### (Comparative Production Example 14)

In a mayonnaise bottle, 40 g of purified water and 10 g of 1,3 butylene glycol were added, and further 50 g of fine titanium oxide particles obtained by treating the surface thereof with hydrogen dimethicone (MT-100SA: particle diameter 15 nm, manufactured by TAYCA CORPORATION) and 100 g of ϕ1.5 mm glass beads were added. After the mixture was dispersed by a paint shaker and the beads were separated, a homogeneous aqueous dispersion having a powder concentration of 50% was obtained.

### (Examples 20 to 25, Comparative Examples 15 to 26: Oil-in-Water Sunscreen)

Oil-in-water sunscreens of Examples 20 to 25 and Comparative Examples 15 to 26 were obtained by using the compositions in Tables 3 and 4 in accordance with the following production method. The unit of the numerical values in the tables is mass%.

### (Production Method)

1. A and B were each heated to 80°C.
2. While B was stirred, A was added and mixed homogeneously.
3. A + B was cooled to 40°C or less.
4. C was added to A + B and mixed homogeneously.
5. D was added to A + B + C and mixed homogeneously.

### <Evaluation Method: Appearance (Dispersion State)>

The dispersion states of the oil-in-water sunscreens obtained in Examples 20 to 25 and Comparative Examples 15 to 26 were visually observed immediately after production and after storage at 45°C for 2 weeks. The state where powder was homogeneously dispersed was evaluated as "∘"; the state where powder aggregation was slightly observed was evaluated as "△"; and the state where powder aggregation was significantly observed was evaluated as "×". The results are shown in Tables 3 and 4.

### <Evaluation Method: Water Repellency>

The oil-in-water sunscreens obtained in Examples 20 to 25 and Comparative Examples 15 to 26 were each applied to an artificial leather using an applicator (gap: 500 µm), and dried at 40°C for 30 minutes to obtain a coating film. To the coating film, 5 µl of water was added dropwise. The contact angle was measured by an automatic contact-angle meter DM-501 manufactured by Kyowa Interface Science Co., Ltd. The results are shown in Tables 3 and 4.

**[Table 3]**

| | Components | Example 20 | Example 21 | Example 22 | Example 23 | Example 24 | Example 25 | Comparative Example 15 | Comparative Example 16 | Comparative Example 17 |
|---|---|---|---|---|---|---|---|---|---|---|
| A | Polyglyceryl-10 stearate | 1.1 | 1.1 | 1.1 | 1.1 | 1.1 | 1.1 | 1.1 | 1.1 | 1.1 |
| | Sorbitan sesquistearate | 0.9 | 0.9 | 0.9 | 0.9 | 0.9 | 0.9 | 0.9 | 0.9 | 0.9 |
| | Cetearyl alcohol | 4.0 | 4.0 | 4.0 | 4.0 | 4.0 | 4.0 | 4.0 | 4.0 | 4.0 |
| | benzoate (C 12-15) | 15.0 | 15.0 | 15.0 | 15.0 | 15.0 | 15.0 | 15.0 | 15.0 | 15.0 |
| | Alkyl | | | | | | | | | |
| | Water | 31.0 | 31.0 | 31.0 | 31.0 | 31.0 | 31.0 | 31.0 | 31.0 | 31.0 |
| B | Propanediol | 5.0 | 5.0 | 5.0 | 5.0 | 5.0 | 5.0 | 5.0 | 5.0 | 5.0 |
| | Sodium methyl stearoyl taurate | 0.5 | 0.5 | 0.5 | 0.5 | 0.5 | 0.5 | 0.5 | 0.5 | 0.5 |
| | | | | | | | | | | |
| C | 2% Xanthan gum aqueous solution (note 1) | 15.0 | - | - | - | - | - | 15.0 | - | - |
| | 1% Hydroxyethyl cellulose aqueous solution (note 2) | - | 15.0 | - | - | - | - | - | 15.0 | - |
| | 2% Polyacrylate cross polymer-6 aqueous solution (note 3) | - | - | 15.0 | - | - | - | - | - | 15.0 |
| | 1% Stearoxyhydroxypropyl methylcellulose aqueous solution (note 4) | - | - | - | 15.0 | - | - | - | - | - |
| | 2% Fluorosilicic acid (Na/Mg) aqueous dispersion (note 5) | - | - | - | - | 15.0 | - | - | - | - |
| | 2% Carbomer aqueous solution (note 6) | - | - | - | - | - | 15.0 | - | - | - |
| | | | | | | | | | | |
| D | Aqueous dispersion of Example 14 | 20.0 | 20.0 | 20.0 | 20.0 | 20.0 | 20.0 | - | - | - |
| | Aqueous dispersion of Comparative Example 13 | - | - | - | - | - | - | 20.0 | 20.0 | 20.0 |
| | Aqueous dispersion of Comparative Example 14 | - | - | - | - | - | - | - | - | - |
| | Water | 7.5 | 7.5 | 7.5 | 7.5 | 7.5 | 7.5 | 7.5 | 7.5 | 7.5 |
| | | 100 | 100 | 100 | 100 | 100 | 100 | 100 | 100 | 100 |
| Evaluation | Total Dispersion state (immediately after) | ○ | ○ | ○ | ○ | ○ | ○ | Δ | Δ | Δ |
| | Dispersion state (45°C, 2 weeks later) | ○ | ○ | ○ | ○ | ○ | ○ | Δ | × | × |
| | Contact angle (°) | 91 | 85 | 82 | 90 | 85 | 90 | 25 | 22 | 28 |

**[Table 4]**

| | Components | Comparative Example 18 | Comparative Example 19 | Comparative Example 20 | Comparative Example 21 | Comparative Example 22 | Comparative Example 23 | Comparative Example 24 | Comparative Example 25 | Comparative Example 26 |
|---|---|---|---|---|---|---|---|---|---|---|
| A | Polyglyceryl-10 stearate | 1.1 | 1.1 | 1.1 | 1.1 | 1.1 | 1.1 | 1.1 | 1.1 | 1.1 |
| | Sorbitan sesquistearate | 0.9 | 0.9 | 0.9 | 0.9 | 0.9 | 0.9 | 0.9 | 0.9 | 0.9 |
| | Cetearvl alcohol | 4.0 | 4.0 | 4.0 | 4.0 | 4.0 | 4.0 | 4.0 | 4.0 | 4.0 |
| | Alkyl benzoate (C12-15) | 15.0 | 15.0 | 15.0 | 15.0 | 15.0 | 15.0 | 15.0 | 15.0 | 15.0 |
| | | | | | | | | | | |
| B | Water | 31.0 | 31.0 | 31.0 | 31.0 | 31.0 | 31.0 | 31.0 | 31.0 | 31.0 |
| | Propanediol | 5.0 | 5.0 | 5.0 | 5.0 | 5.0 | 5.0 | 5.0 | 5.0 | 5.0 |
| | Sodium methyl stearovl taurate | 0.5 | 0.5 | 0.5 | 0.5 | 0.5 | 0.5 | 0.5 | 0.5 | 0.5 |
| | | | | | | | | | | |
| C | 2% Xanthan gum aqueous solution (note 1) | - | - | - | 15.0 | - | - | - | - | - |
| | 1% Hydroxyethyl cellulose aqueous solution (note 2) | - | - | - | - | 15.0 | - | - | - | - |
| | 2% Polyacrylate cross polymer-6 aqueous solution (note 3) | - | - | - | - | - | 15.0 | - | - | - |
| | 1% Stearoxyhydroxypropyl methylcellulose aqueous solution (note 4) | 15.0 | - | - | - | - | - | 15.0 | - | - |
| | 2% Fluorosilicic acid (Na/Mg) aqueous dispersion (note 5) | - | 15.0 | - | - | - | - | - | 15.0 | - |
| | 2% Carbomer aqueous solution (note 6) | - | - | 15.0 | - | - | - | - | - | 15.0 |
| | | | | | | | | | | |
| D | Aqueous dispersion of Example 14 | - | - | - | - | - | - | - | - | - |
| | Aqueous dispersion of Comparative Example 13 | 20.0 | 20.0 | 20.0 | - | - | - | - | - | - |
| | Aqueous dispersion of Comparative Example 14 | - | - | - | 25.0 | 25.0 | 25.0 | 25.0 | 25.0 | 25.0 |
| | Water | 7.5 | 7.5 | 7.5 | 2.5 | 2.5 | 2.5 | 2.5 | 2.5 | 2.5 |
| | Total | 100 | 100 | 100 | 100 | 100 | 100 | 100 | 100 | 100 |
| Evaluation | Dispersion state (immediately after) | Δ | Δ | × | ○ | × | × | × | × | × |
| | Dispersion state (45°C, 2 weeks later) | × | Δ | × | ○ | × | × | × | × | × |
| | Contact angle (°) | 26 | 30 | 25 | 15 | 10 | 15 | 17 | 15 | 13 |

(note 1): Echo Gum T (CP Kelco U.S., Inc.)
(note 2): HEC Daicel SE900 (manufactured by Daicel Miraizu Ltd.)
(note 3): SEPIMAX ZEN (manufactured by SEPPIC S.A.)
(note 4): SANGELOSE 90 L (manufactured by Daido Chemical Corporation)
(note 5): Laponite-XL21 XR (manufactured by BYK Japan KK)
(note 6): NTC-CARBOMER 381 (manufactured by Guangzhou Tinc silicone technology)

From the results shown in Tables 3 and 4, it is apparent that the cosmetic of the invention has a satisfactory dispersion state immediately after production and after storage at 45°C for 2 weeks, and has high water repellency (high water resistance). In addition, the cosmetics of Examples had excellent feeling in use such as freshness. Water freshness was evaluated as ∘ in the above evaluation method.

### (Example 26: Gel Sunscreen)

### (Component) (%)

| | |
|---|---|
| 1. Purified Water | 28.0 |
| 2. Propanediol | 7.0 |
| 3. 1% Stearoxyhydroxypropyl methylcellulose aqueous solution (note 7) | 40.0 |
| 4. Aqueous dispersion of Example 15 | 15.0 |
| 5. Silica (note 8) | 5.0 |
| 6. Purified Water | 5.0 |

| | |
|---|---|
| (note 7): SANGELOSE 90 L (manufactured by Daido Chemical Corporation) (note 8): Sunsphere NP-30 (manufactured by AGC Si-Tech Co., Ltd.) | |

### (Production Method)

A: Components 1 and 2 were mixed and homogenized.
B: Component 3 was added to A, mixed, and homogenized.
C: Component 4 was added to B, mixed, and homogenized.
D: Component 5 and Component 6 were homogeneously mixed, then added to C, mixed, and homogenized to obtain a gel sunscreen.

The gel sunscreen obtained as described above spread easily, provided a fresh and refreshing feeling of use. Furthermore, makeup lasted satisfactorily long. Moreover water resistance thereof was high and stability was excellent without aggregation or sedimentation of powder over time.

### (Example 27: Gel Sunscreen)

### (Component) (%)

| | |
|---|---|
| 1. Purified Water | 66.0 |
| 2. Propanediol | 7.0 |
| 3. 1% Stearoxyhydroxypropyl methylcellulose aqueous solution (note 9) | 2.0 |
| 4. Aqueous dispersion of Example 15 | 15.0 |
| 5. Silica (note 10) | 5.0 |
| 6. Purified water | 5.0 |

| | |
|---|---|
| (note 9): SANGELOSE 90 L (manufactured by Daido Chemical Corporation) (note 10): Sunsphere NP-30 (manufactured by AGC Si-Tech Co., Ltd.) | |

### (Production Method)

A gel sunscreen was obtained by the same production method as in Example 26.

The gel sunscreen obtained as described above spread easily, provided a fresh and refreshing feeling of use. Furthermore, makeup lasted satisfactorily long. Moreover water resistance thereof was high and stability was excellent without aggregation or sedimentation of powder over time.

### (Example 28: Gel Sunscreen)

### (Component) (%)

| | |
|---|---|
| 1. Propanediol | 5.0 |
| 2. 2.5% Polyacrylate cross polymer-6 aqueous solution (note 11) | 80.0 |
| 3. Aqueous dispersion of Example 15 | 15.0 |

| | |
|---|---|
| (note 11): SEPIMAX ZEN (manufactured by SEPPIC S.A.) | |

### (Production Method)

All components were mixed and homogenized.

The gel sunscreen obtained as described above spread easily, provided a fresh and refreshing feeling of use. Furthermore, makeup lasted satisfactorily long. Moreover water resistance thereof was high and stability was excellent without aggregation or sedimentation of powder over time.

### (Example 29: Oil-in-Water Type Makeup Base)

### (Component) (%)

| | |
|---|---|
| 1. Behenyl alcohol | 1.3 |
| 2. Polyglyceryl-10 pentastearate | 1.0 |
| 3. Sodium stearoyl lactylate | 0.2 |
| 4. Cetyl ethylhexanoate | 15.0 |
| 5. Ethylhexyl methoxycinnamate | 5.0 |
| 6. Purified Water | 39.5 |
| 7. Propanediol | 5.0 |
| 8. 2% Polyacrylate cross polymer-6 aqueous solution (note 12) | 25.0 |
| 9. Aqueous dispersion of Example 16 | 6.7 |
| 10. Aqueous dispersion of Example 17 | 0.2 |
| 11. Aqueous dispersion of Example 18 | 1.0 |
| 12. Aqueous dispersion of Example 19 | 0.1 |

| | |
|---|---|
| (note 12) SEPIMAX ZEN (manufactured by SEPPIC S.A.) | |

### (Production Method)

A: Components 1 to 5 were heated to 80°C, mixed and homogenized.
B: Component 6 and component 7 were heated to 80°C, mixed and homogenized.
C: A was added to B while stirring B, mixed, and homogenized.
D: Component 8 is added to C, mixed, and homogenized.
E: Components 9 to 12 were added to D, mixed, and homogenized to obtain an oil-in-water type makeup base.

The oil-in-water type makeup base obtained as described above had fine texture, satisfactory spreadability without stickiness or greasiness. Furthermore, the makeup base was moist and fresh, and provides refreshing feeling in use and simultaneously made makeup to last satisfactorily long. Moreover, water resistance thereof was high without aggregation or sedimentation of powder over time.

The present application is based on JP Application 2022-182769 filed on November 15, 2022 and JP Application 2023-149732 filed on September 15, 2023, the disclosures of which are incorporated herein by reference in their entireties.

## Claims

1. An aqueous dispersion of an inorganic powder with a hydrophobized surface, comprising the following components (A), (B), and (C) as essential components:
(A) inorganic powder with a hydrophobized surface;
(B) a dialkylsulfosuccinate and/or an alkylsulfoacetate; and
(C) water.

2. The aqueous dispersion of an inorganic powder with a hydrophobized surface according to claim 1, wherein component (A) is an inorganic powder with a hydrophobized surface with at least one compound selected from the group consisting of silicone oil, an alkoxysilane, and a fatty acid.

3. A cosmetic comprising the aqueous dispersion of an inorganic powder with a hydrophobized surface according to claim 1 or 2.

4. The cosmetic according to claim 3, further comprising a water-soluble polymer and/or an inorganic thickener as component (D).

5. The cosmetic according to claim 4, wherein a content of component (D) is 0.02 mass% to 2 mass%.

6. A method for producing a cosmetic, comprising mixing the aqueous dispersion of an inorganic powder having hydrophobically treated in surface according to claim 1 or 2 with a cosmetic component.
